# EUROPEAN PATENT SPECIFICATION

(11) **EP 2 493 559 B1**
(45) Date of publication and mention of the grant of the patent: **30.03.2016**
(21) Application number: 11730161.4
(22) Date of filing: 29.06.2011
(51) Int. Cl.: A61N 1/36

(54) **TWO-PIECE SOUND PROCESSOR SYSTEM FOR USE IN AN AUDITORY PROSTHESIS SYSTEM**
ZWEITEILIGES KLANGPROZESSORSYSTEM ZUR VERWENDUNG IN EINEM HÖRPROTHESENSYSTEM
SYSTÈME DE PROCESSEUR DE SON EN DEUX PARTIES À UTILISER DANS UN SYSTÈME DE PROTHÈSE AUDITIVE

(43) Date of publication of application: 05.09.2012
(73) Proprietor: Advanced Bionics AG, 8712 Stäfa (CH)
(72) Inventor: MISHRA, Lakshmi N., Valencia, California 91354 (US); CHEN, Joey, Valencia, California 91355 (US)
(74) Representative: Schwan Schorer & Partner mbB
(86) International application number: PCT/US2011/042286
(87) International publication number: WO 2012/099620

(56) References cited:
- WO-A1-02/056637
- US-A- 5 824 022
- US-A1- 2011 098 786
- SOON KWAN AN ET AL: "Design for a Simplified Cochlear Implant System", IEEE TRANSACTIONS ON BIOMEDICAL ENGINEERING, IEEE SERVICE CENTER, PISCATAWAY, NJ, USA, vol. 54, no. 6, 1 June 2007 (2007-06-01), pages 973-982, XP011181881, ISSN: 0018-9294, DOI: 10.1109/TBME.2007.895372

## Description

### BACKGROUND INFORMATION

In the field of auditory prosthesis systems (e.g., cochlear implant systems), there are often two competing requirements - size and performance. For example, auditory prosthesis users would like the smallest devices possible while simultaneously being able to connect to auxiliary audio input devices (e.g., FM transmitters, Bluetooth devices, audio players, etc.), manually adjust various control parameters (e.g., volume, sensitivity, etc.), and/or otherwise enjoy a variety of other features that many auditory prosthesis systems have to offer. Unfortunately, many of these features require specialized hardware (e.g., external interface components, user input mechanisms, etc.) and consume a relatively large amount of power. As a result, the hardware that the user has to wear (e.g., behind or on his or ear) is relatively large, bulky, and aesthetically unpleasing.

US 2011/0098786 A1 relates to a cochlear implant system comprising a BTE (behind-the-ear) sound processing unit, an implanted auditory prosthesis and a remote audio processor module, wherein at least a portion of the signal processing heuristic may be performed by the remote audio processor module which is selectively and communicatively coupled to the BTE sound processing unit a communication link. The remote audio processor module may be selectively removed from being communicatively coupled to the BTE sound processing unit. A headpiece fixed to the patient's heas and housing a coil communicating with a respective coil in the implanted auditory prosthesis is provided separate from the BTE sound processing unit.

### SUMMARY

The invention relates to a system as defined in claim 1. An exemplary system includes 1) a headpiece module configured to be affixed to a head of a patient and comprising a primary sound processor configured to generate stimulation parameters used to direct an auditory prosthesis implanted within the patient to apply electrical stimulation representative of one or more audio signals to the patient and 2) a sound processor module separate from the headpiece module and configured to be selectively and communicatively coupled to the headpiece module. The sound processor module includes a secondary sound processor configured to detect a communicative coupling of the sound processor module to the headpiece module and contribute to the generation of one or more of the stimulation parameters while the sound processor module is communicatively coupled to the headpiece module.

An exemplary headpiece module is configured to be affixed to a head of a patient and includes 1) a primary sound processor configured to generate stimulation parameters used to direct an auditory prosthesis implanted within the patient to apply electrical stimulation representative of one or more audio signals to the patient and 2) communication circuitry communicatively coupled to the primary sound processor configured to transmit the stimulation parameters to the auditory prosthesis. The primary sound processor is further configured to detect a communicative coupling of a sound processor module comprising a secondary sound processor to the headpiece module and allow the secondary sound processor to contribute to the generation of one or more stimulation parameters included in the stimulation parameters while the sound processor module is communicatively coupled to the headpiece module.

An exemplary method includes a primary sound processor included in a headpiece module 1) generating stimulation parameters used to direct an auditory prosthesis implanted within the patient to apply electrical stimulation representative of one or more audio signals to the patient, 2) detecting a communicative coupling of a sound processor module to the headpiece module, the sound processor module separate from the headpiece module and comprising a secondary sound processor, and 3) allowing the secondary sound processor to contribute to the generation of one or more stimulation parameters included in the stimulation parameters while the sound processor module is communicatively coupled to the headpiece module.

### DETAILED DESCRIPTION

A two-piece sound processor system for use in an auditory prosthesis system is described herein. As will be described in more detail below, the two-piece sound processor system may include a primary sound processor located within a headpiece module configured to be affixed to a head of a patient (or user) and a secondary sound processor located within a sound processor module configured to be worn or carried by the user behind the ear, on the ear, or at any other suitable location. The sound processor module may be configured to be selectively and communicatively coupled to the headpiece module by way of a wired and/or wireless communication link.

In some examples, the primary sound processor may independently generate stimulation parameters used to direct an auditory prosthesis implanted within the patient to apply electrical stimulation representative of one or more audio signals to the patient. However, during periods in which the sound processor module is communicatively coupled to the headpiece module, the secondary sound processor may contribute to the generation of one or more of the stimulation parameters. For example, the secondary sound processor may apply one or more pre-processing functions to an audio signal and then transmit data representative of the pre-processed audio signal to the primary sound processor for further processing, provide connectivity to one or more external devices (e.g., auxiliary audio input devices, fitting devices, etc.), facilitate user adjustment of one or more of the stimulation parameters (e.g., by providing one or more user input mechanisms with which the user may interact), and/or otherwise provide one or more sound processing features not available with the primary sound processor alone. In this manner, the patient may wear the headpiece module only (and thereby enjoy the benefits of not having to wear a sound processor unit on or behind his or her ear) and still be able to enjoy basic auditory prosthesis functionality. When the patient wishes to access additional sound processing features not available with the primary sound processor alone, the patient may simply connect the sound processor module to the headpiece module (e.g., by putting the sound processor module on).

According to a first embodiment an exemplary auditory prosthesis system may include a headpiece module, a sound processor module, and an auditory prosthesis. The headpiece module may include a primary sound processor disposed therein and the sound processor module may include a secondary sound processor therein. The auditory prosthesis may be communicatively coupled to a lead having a plurality of electrodes disposed thereon. Each of these components will now be described in more detail.

The headpiece module may be configured to be affixed to a patient's head and positioned such that communication circuitry (e.g., a coil) housed within the headpiece module is communicatively coupled to corresponding communication circuitry (e.g., a coil) included within the auditory prosthesis. In this manner, the headpiece module may be communicatively coupled to the auditory prosthesis in a wireless manner by a communication link. The Headpiece module may be of any suitable shape, size, and form factor as may serve a particular implementation. Various components and features of the headpiece module will be described below.

The sound processor module 104 may include any suitable device configured to be worn behind the ear of a patient (i.e., a behind-the-ear device) or off the ear (i.e., a body worn device) of the patient. An exemplary off-the-ear sound processor module may be secured to a piece of clothing worn by the patient, carried in a pocket or pouch, and/or otherwise carried by the patient. Various components and features of the sound processor module will be described below.

In some examples, the sound processor module may be selectively and communicatively coupled to the headpiece module by way of a communication link, which may be wired (e.g., implemented by one or more wires, cables, etc.) or wireless as may serve a particular implementation. To illustrate, the sound processor module may be selectively and communicatively coupled to the headpiece module by plugging the sound processor in to the headpiece module, placing the sound processor module within range of the headpiece module (e.g., by placing the sound processor module 104 behind or on the ear of a patient), turning "on" the sound processor module 104, etc.

The auditory prosthesis may be partially or fully implanted within a patient and configured to apply electrical stimulation representative of one or more audio signals by way of one or more of the electrodes to one or more stimulation sites associated with an auditory pathway (e.g., the auditory nerve) of the patient. In some examples, the auditory prosthesis may apply the electrical stimulation in accordance with stimulation parameters provided thereto by primary sound processor. The auditory prosthesis may include any type of implantable stimulator (e.g., cochlear implant, brainstem implant, etc.) that may be used in association with the systems and methods described herein.

The primary sound processor located within the headpiece module and the secondary sound processor located within the sound processor module may together be referred to herein as a "two-piece sound processor system" and may each include or be implemented by any combination of hardware, software, and/or firmware as may serve a particular implementation. For example, the primary sound processor and/or the secondary sound processor may each include or be implemented by a computing device or processor configured to perform one or more of the functions described herein. Exemplary components and features of the primary sound processor and the secondary sound processor will now be described.

The primary sound processor may include a communication facility, a detection facility, a processing facility, and a storage facility, which may be in communication with one another using any suitable communication technologies. These facilities will now be described in more detail.

The communication facility may be configured to facilitate communication between the primary sound processor and the secondary sound processor and between the primary sound processor and the auditory prosthesis. For example, the communication facility may include one or more components configured to receive data from the secondary sound processor and/or transmit stimulation parameters to the auditory prosthesis.

The detection facility is configured to detect a communicative coupling of the sound processor module to the headpiece module. For example, the detection facility may detect that the sound processor module has been plugged in to the headpiece module, placed within range of the headpiece module, turned on, etc. As will be described below, the primary sound processor is configured to allow the secondary sound processor to contribute to the generation of one or more stimulation parameters while the sound processor module is communicatively coupled to the headpiece module.

The detection facility may be further configured to detect a communicative decoupling of the sound processor module from the headpiece module. For example, the detection facility may detect that the sound processor module has been unplugged from the headpiece module, removed from being placed within range of the headpiece module, turned off, etc. As will be described below, the primary sound processor may be configured to generate stimulation parameters independent of the secondary sound processor while the sound processor module is not communicatively coupled to the headpiece module.

The processing facility may be configured to perform one or more sound processing operations on one or more audio signals presented to an auditory prosthesis patient. For example, the processing facility may generate stimulation parameters used to direct the auditory prosthesis to apply electrical stimulation representative of one or more audio signals to the patient. One or more of the stimulation parameters may be generated by the processing facility independent of the secondary sound processor. Additionally or alternatively, one or more of the stimulation parameters may be generated by the processing facility in conjunction with the secondary sound processor. For example, the processing facility may generate, adjust, and/or otherwise modify one or more stimulation parameters based on data transmitted to the primary sound processor by the secondary sound processor.

The processing facility may be configured to perform any other signal processing operation as may serve a particular implementation. For example, the processing facility may detect one or more audio signals presented to a patient, adjust one or more stimulation parameters, and/or transmit one or more stimulation parameters to the auditory prosthesis.

The storage facility may be configured to maintain the stimulation parameter data representative of one or more stimulation parameters that may be transmitted to the auditory prosthesis. The storage facility may be configured to maintain additional or alternative data as may serve a particular implementation.

The secondary sound processor may include a communication facility, a detection facility, a processing facility, an interface facility, and a storage facility, which may be in communication with one another using any suitable communication technologies. These facilities will now be described in more detail.

The communication facility may be configured to facilitate communication between the secondary sound processor and the primary sound processor. For example, the communication facility may include one or more components configured to transmit data to and/or receive data from the primary sound processor. Exemplary data that may be transmitted by the communication facility to the primary sound processor includes data representative of a pre-processed audio signal, fitting data, auxiliary audio input data, etc.

The detection facility may be configured to detect a communicative coupling of the sound processor module to the headpiece module. For example, the detection facility may detect that the sound processor module has been plugged in to the headpiece module, placed within range of the headpiece module, turned on, etc. As mentioned, the secondary sound processor may be configured to contribute to the generation of one or more stimulation parameters while the sound processor module is communicatively coupled to the headpiece module.

The processing facility may be configured to contribute to the generation of one or more stimulation parameters by the primary sound processor while the sound processor module is communicatively coupled to the headpiece module. For example, as will be illustrated below, the sound processor module may include one or more user input mechanisms (e.g., a program selection mechanism, a volume control mechanism, and/or a sensitivity control mechanism) with which a user (e.g., the patient) may interact. The processing facility may detect interaction by a user with one or more user input mechanisms and, in response, adjust one or more stimulation parameters (e.g., by transmitting data to the primary sound processor directing the primary sound processor to adjust the one or more stimulation parameters).

Additionally or alternatively, the processing facility may be configured to contribute to the generation of one or more stimulation parameters by detecting an audio signal presented to a patient, applying one or more pre-processing functions to the audio signal, and transmitting data representative of the pre-processed audio signal to the primary sound processor. The processing facility may apply a pre-processing function to an audio signal by amplifying the audio signal, converting the audio signal to a digital signal, filtering data representative of the audio signal with a pre-emphasis filter, subjecting data representative of the audio signal to automatic gain control, performing a beam forming function associated with the detection of the audio signal, and/or in any other manner as may serve a particular implementation.

Additionally or alternatively, the processing facility may be configured to contribute to the generation of one or more stimulation parameters by processing and/or transmitting data representative of an audio signal provided by an auxiliary audio input device to the primary sound processor for further processing. As mentioned, an auxiliary audio input device may include a FM transmitter, a Bluetooth device, an audio player (e.g., an MP3 player), and/or any other type of device configured to provide auxiliary audio input.

The storage facility may be configured to maintain audio content data representative of audio content detected or otherwise received by the secondary sound processor, pre-processing data representative pre-processing data to be transmitted from the secondary sound processor to the primary sound processor, and/or any other type of data as may serve a particular implementation. The storage facility may be configured to maintain additional or alternative data as may serve a particular implementation.

Additional components may be included in the headpiece module and additional components may be included in or connected to the sound processor module. It will be recognized that the additional components described hereinafter are merely illustrative of the many different components that may be included in or otherwise connected to the headpiece module and/or the sound processor module as may serve a particular implementation. The headpiece module additionally includes a battery.

The headpiece module may additionally include a microphone, and communication circuitry. Each of these components may be communicatively coupled to the primary sound processor in any suitable manner.

The battery may include any suitable type of battery and may be configured to provide power to one or more components within the headpiece module. The battery may additionally or alternatively be configured to provide power to the auditory prosthesis. For example, power derived from the battery may be transmitted to auditory prosthesis by way of the communication link. The battery is additionally or alternatively configured to provide power to one or more components included in the sound processor module. Operating power derived from the battery is transmitted to the secondary sound processor by way of the communication link while the sound processor module is communicatively coupled to the headpiece module. In this manner, the sound processor module does not need to include its own separate battery.

The microphone may be configured to receive (e.g., detect) one or more audio signals presented to a patient for processing by the primary sound processor. The microphone may be at least partially disposed within the headpiece module. Alternatively, the microphone may be separate from the headpiece module and communicatively coupled to one or more components within the headpiece module as may serve a particular implementation.

The communication circuitry may include any suitable combination of components (e.g., one or more coils, transceivers, etc.) configured to facilitate transmission of data in between the headpiece module and the auditory prosthesis and/or in between the headpiece module and the sound processor module. For example, the communication circuitry may be configured to transmit stimulation parameters generated by the primary sound processor to the auditory prosthesis. Additionally or alternatively, the communication circuitry may be configured to receive data from the secondary sound processor for processing by the primary sound processor.

The sound processor module may additionally include one or more user input mechanisms and one or more interface components. The sound processor module may additionally be configured to be selectively and communicatively coupled to an external device and one or more microphones. The user input mechanisms, the interface components, the external device, and the microphones may be communicatively coupled to the secondary sound processor in any suitable manner as may serve a particular implementation.

The user input mechanisms may include one or more mechanisms with which a user (e.g., an auditory prosthesis patient) may interact to provide user input to the secondary sound processor and/or the primary sound processor. For example, the user input mechanisms may include a program selection mechanism (e.g., a program selection switch) configured to allow a user to manually direct the primary sound processor to switch from operating in accordance with a particular sound processing program to operating in accordance with a different sound processing program. The user input mechanisms may additionally or alternatively include a volume control mechanism (e.g., a volume control knob configured to allow a user to adjust one or more volume levels, a sensitivity control mechanism (e.g., a sensitivity control knob) configured to allow a user to adjust one or more sensitivity levels, and/or any other type of user input mechanism as may serve a particular implementation.

The interface components may include any suitable combination of components configured to interface with one or more external devices (i.e., facilitate communication by the secondary sound processor and/or the primary sound processor with the one or more external devices). For example, the interface components may include in or more input ports, communication components, etc. configured to facilitate communicative coupling of the sound processor module to the one or more external devices.

To illustrate, the interface components may interface with the external device. The external device may include an auxiliary audio input device, a fitting device, a clinician's programming interface device, a hearing instrument body area network ("HiBAN") device, an audio gateway device (e.g., iCOM), a remote control device (e.g., myPilot), or another sound processor (e.g., another sound processor used in a bilateral auditory prosthesis system).

As an example, the external device may include an auxiliary audio input device, such as an audio player. To experience audio presented by the auxiliary audio input device, a user may couple the sound processor module to the headpiece module and then couple the auxiliary audio input device to the sound processor module (or in reverse order). The secondary sound processor may then receive audio signals from the auxiliary audio input device by way of the interface components and transmit data representative of the received audio signals to the primary sound processor. In some examples, the secondary sound processor may apply one or more pre-processing functions to the audio signals before they are transmitted to the primary sound processor.

As another example, the external device may include a fitting device that may be used by an audiologist or the like to fit the primary sound processor to a patient. To this end, the sound processor module may be coupled to the headpiece module. Fitting data may then be relayed from the fitting device to the primary sound processor by the secondary sound processor.

The multiple microphones may be communicatively coupled to the sound processor module. The microphones may be placed at any suitable location (e.g., within the opening of the ear). The multiple microphones may facilitate performance of one or more beam forming functions by the secondary sound processor, which may increase the overall performance of the auditory prosthesis system. It will be recognized that any number of microphones may be communicatively coupled to the sound processor module.

In an exemplary physical implementation of the auditory prosthesis system. The headpiece module includes a housing within which various components, including the primary sound processor, may be disposed. A microphone may be integrated into a surface of the housing.

The sound processor module includes a device configured to be worn or carried by the patient off the ear. Alternatively, as described above, the sound processor may include a behind-the-ear device. The sound processor module includes a housing within which various components, including the secondary sound processor, may be disposed. User input mechanisms (e.g., volume and sensitivity control knobs) and an interface port (e.g., an auxiliary audio input port) may be integrated into a surface of the housing.

The sound processor module may be selectively coupled to the headpiece module. For example, the sound processor module may be coupled to the headpiece module by way of a cable. To this end, the sound processor module and the headpiece module may each include a connector port (not shown) configured to connect to corresponding connectors of the cable. When a user desires to disconnect the sound processor module from the headpiece module, he or she may remove a connector of the cable from the connector port of the sound processor module and/or a connector of the cable from the connector port of headpiece module. It will be recognized that the sound processor module may additionally or alternatively be configured to wirelessly connect to the headpiece module.

According to another exemplary implementation of the auditory prosthesis system the sound processor module is configured to provide electro-acoustic stimulation functionality to a user of the headpiece module. As used herein, "electro-acoustic stimulation" refers to the use of a hearing aid and an auditory prosthesis (e.g., a cochlear implant) together in the same ear. The hearing aid acoustically amplifies the low frequencies while the auditory prosthesis electrically stimulates the high frequencies. The auditory nerve combines the acoustic and electric stimuli to one auditory signal.

To this end, a microphone and a loudspeaker may be communicatively coupled to the sound processor module. This microphone may be similar in function to the multiple microphones mentioned above. The loudspeaker (also referred to as a receiver) may be configured to apply acoustic stimulation to the user. For example, at the direction of the secondary sound processor, the loudspeaker may present an amplified version of audio content included in a low frequency band to the user.

To illustrate, the secondary sound processor may detect audio content (e.g., one or more audio signals) presented to the user by way of the microphone. The secondary sound processor may then determine whether the detected audio content is included in a "high" frequency band (e.g., a frequency band substantially equal to 1 kHz - 8 kHz) or in a "low" frequency band (e.g., a frequency band substantially equal to 100 Hz - 1 kHz). It will be recognized that the particular frequencies associated with the high and low frequency bands may vary as may serve a particular implementation.

If the audio content is included in the high frequency band, the secondary sound processor may transmit data representative of the audio content to the primary sound processor or otherwise instruct the primary sound processor to direct the auditory prosthesis to apply electrical representative of the audio content to the user. Alternatively, if the audio content is included in the low frequency band, the secondary sound processor may direct the loudspeaker to apply acoustic stimulation representative of the audio content to the patient.

It will be recognized that the electro-acoustic stimulation functionality of the sound processor module may be combined with the functionality of the sound processor module described above. The sund processor module may provide additional or alternative functionality as may serve a particular implementation.

Hereinafter an exemplary method in accordance with the systems will be described. While exemplary steps according to one embodiment will be described hereinafter, other embodiments may omit, add to, reorder, and/or modify any of these steps. One or more of these steps may be performed by any component or combination of components of primary sound processor.

In a first step, a primary sound processor included in a headpiece module generates stimulation parameters used to direct an auditory prosthesis implanted within the patient to apply electrical stimulation representative of one or more audio signals to the patient. The first step may be performed in any of the ways described herein.

In a second step, the primary sound processor detects a communicative coupling of a sound processor module to the headpiece module. The sound processor module is separate from the headpiece module and comprises a secondary sound processor. The second step may be performed in any of the ways described herein.

In a third step, the primary sound processor allows the secondary sound processor to contribute to the generation of one or more stimulation parameters included in the stimulation parameters while the sound processor module is communicatively coupled to the headpiece module. The third step may be performed in any of the ways described herein.

In the preceding description, various exemplary embodiments have been described. It will, however, be evident that various modifications and changes may be made thereto, and additional embodiments may be implemented, without departing from the scope of the invention as set forth in the claims that follow. For example, certain features of one embodiment described herein may be combined with or substituted for features of another embodiment described herein. The description is accordingly to be regarded in an illustrative rather than a restrictive sense.

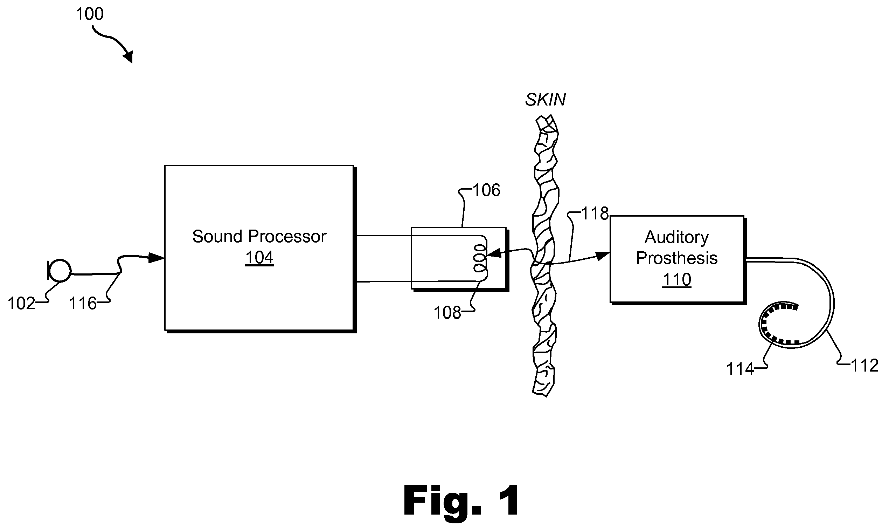

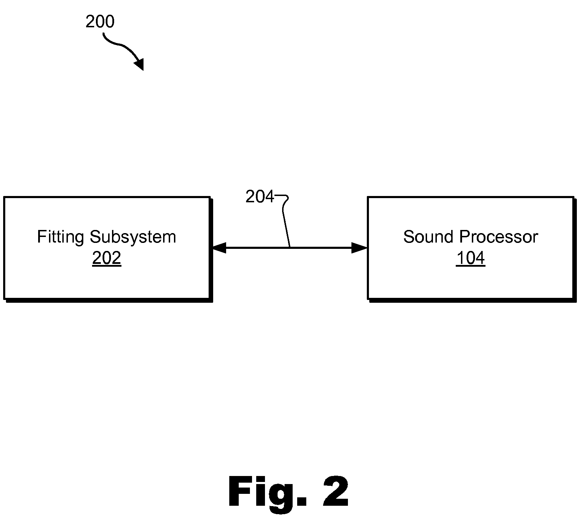

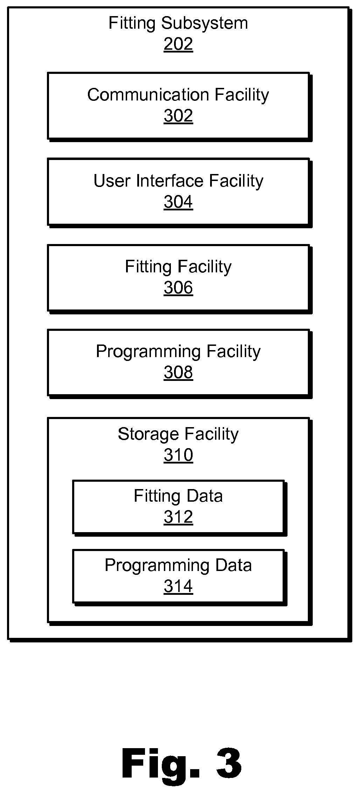

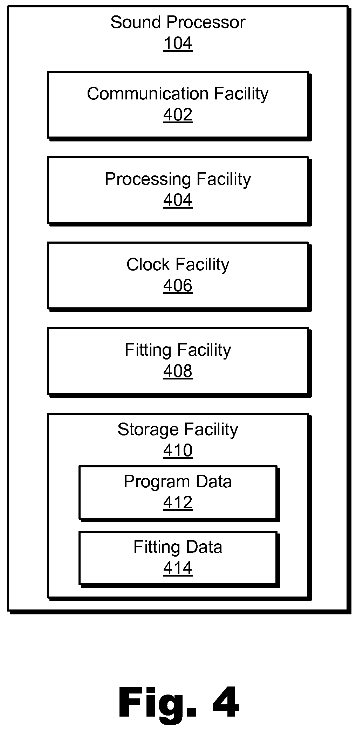

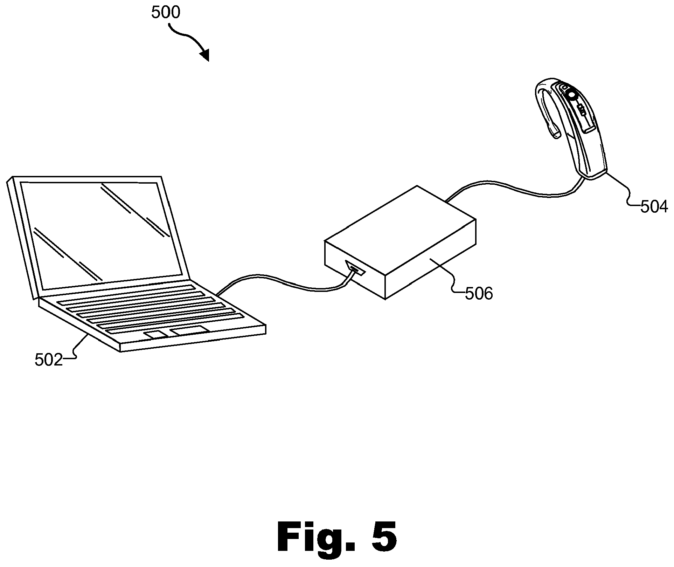

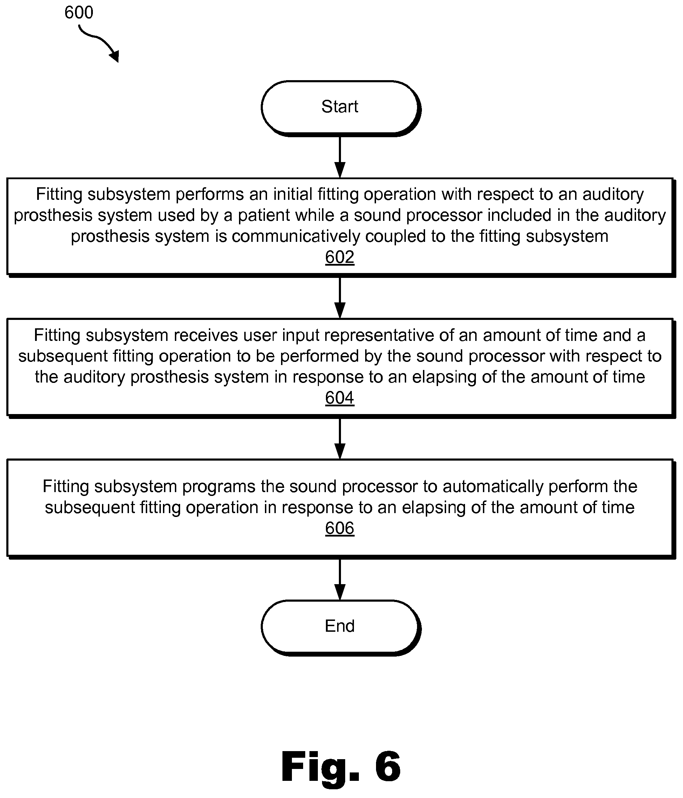

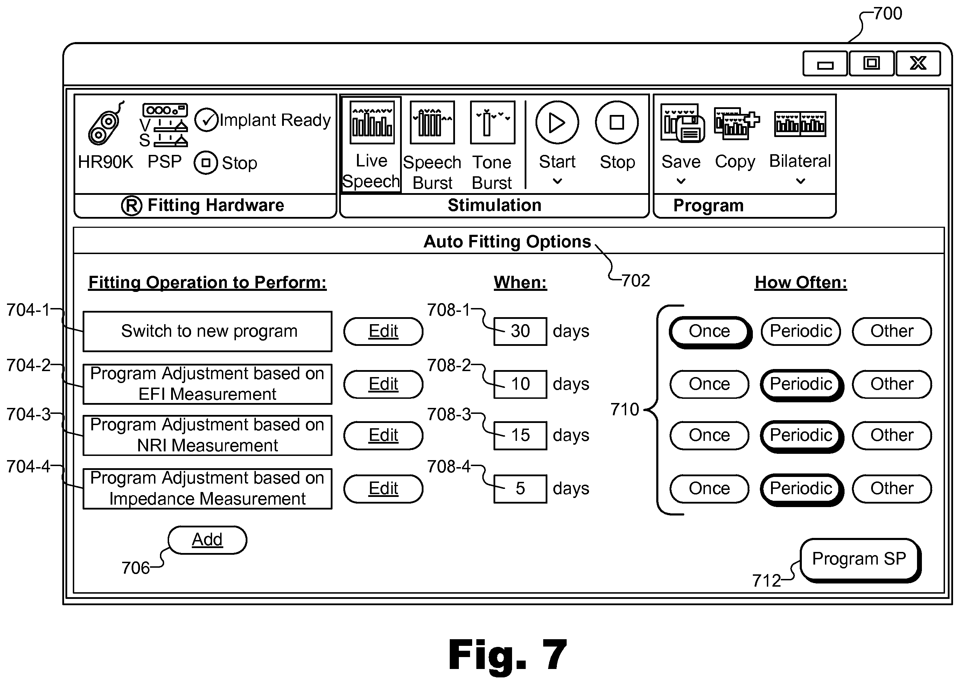

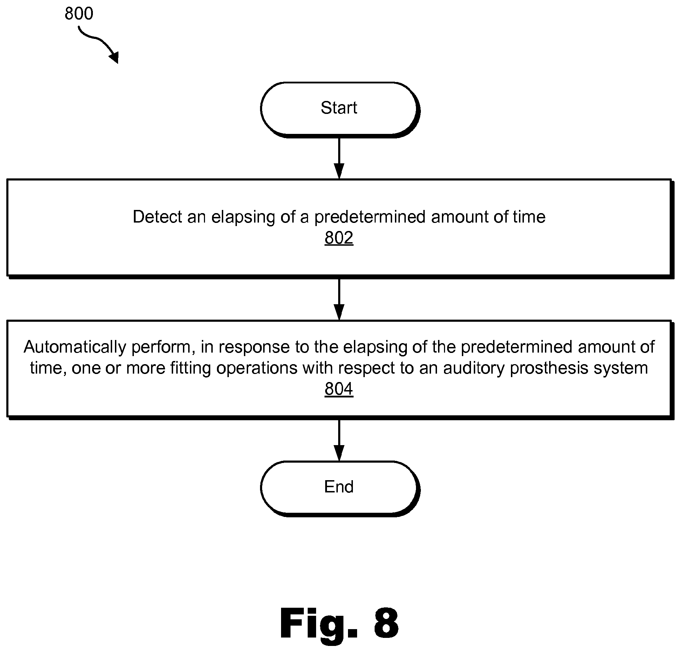

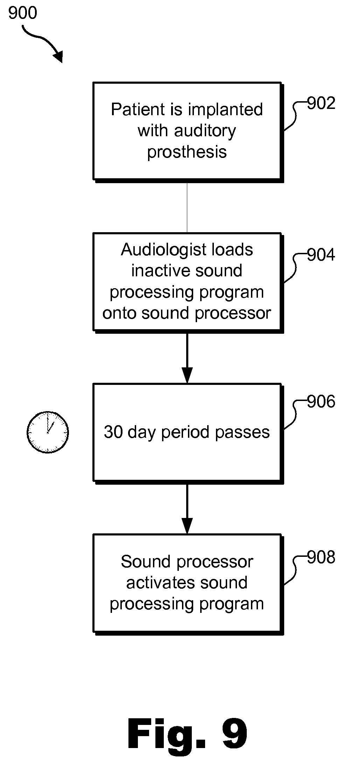

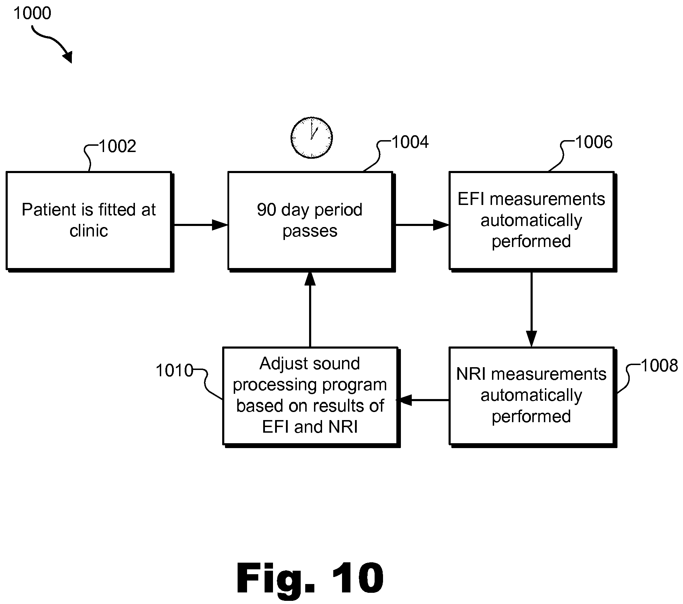

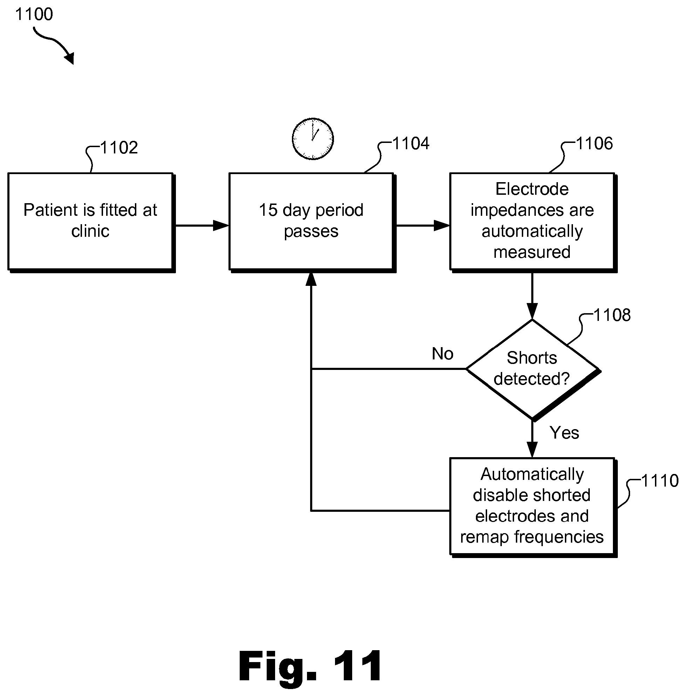

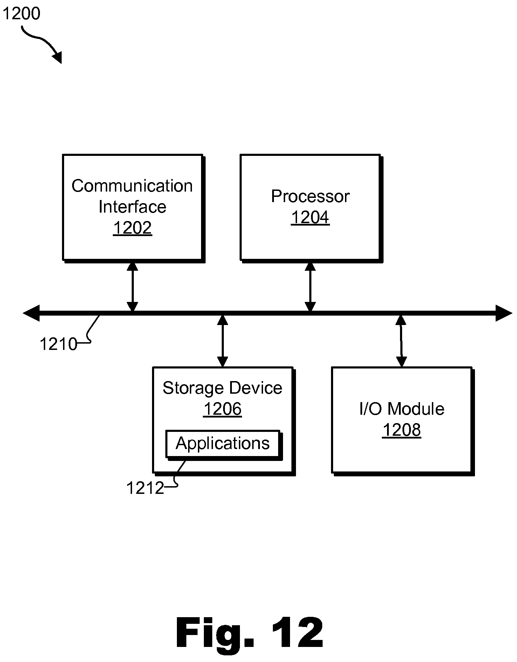

## Claims

1. A system comprising:
a headpiece module configured to be affixed to a head of a patient and comprising a primary sound processor disposed therein that is configured to generate stimulation parameters used to direct an auditory prosthesis implanted within the patient to apply electrical stimulation representative of one or more audio signals to the patient and a battery the headpiece module housing communication circuitry that is wirelessly communicatively coupled to communication circuitry included within the auditory prosthesis; and
a sound processor module which is separate from the headpiece module, and which is configured to be selectively and communicatively coupled to the headpiece module, the sound processor module comprising a secondary sound processor configured to
detect a communicative coupling of the sound processor module to the headpiece module, and
contribute to the generation of one or more stimulation parameters included in the stimulation parameters while the sound processor module is communicatively coupled to the headpiece module,
wherein the primary sound processor is configured to
detect that the sound processor module is not communicatively coupled to the headpiece module; and
generate the stimulation parameters independent of the secondary sound processor while the sound processor module is not communicatively coupled to the headpiece module,
**characterized in that**
the primary sound processor is configured to provide, while the sound processor module is communicatively coupled to the headpiece module, operating power to the secondary sound processor.

2. The system of claim 1, wherein the primary sound processor is further configured to generate one or more of the stimulation parameters independent of the secondary sound processor while the sound processor module is communicatively coupled to the headpiece module.

3. The system of claim 1, wherein the sound processor module comprises one or more user input mechanisms, and wherein the secondary sound processor is configured to contribute to the generation of the one or more stimulation parameters by
detecting interaction by a user with the one or more user input mechanisms; and
adjusting the one or more stimulation parameters in response to the interaction by the user with the one or more user input mechanisms.

4. The system of claim 3, wherein the one or more user input mechanisms comprise at least one of a program selection mechanism, a volume control mechanism, and a sensitivity control mechanism.

5. The system of claim 1, wherein the one or more stimulation parameters are associated with an audio signal included in the one or more audio signals, and wherein the secondary sound processor is configured to contribute to the generation of the one or more stimulation parameters by:
detecting the audio signal;
applying one or more pre-processing functions to the audio signal; and
transmitting data representative of the pre-processed audio signal to the primary sound processor.

6. The system of claim 5, wherein the sound processor module is communicatively coupled to one or more microphones, and wherein the secondary sound processor is configured to detect the audio signal using the one or more microphones.

7. The system of claim 6, wherein the one or more pre-processing functions comprises a beam forming function associated with the detection of the audio signals using the one or more microphones.

8. The system of claim 1, wherein the sound processor module further comprises one or more interface components configured to facilitate communication by the secondary sound processor with one or more auxiliary audio input devices, and wherein the secondary sound processor is configured to contribute to the generation of the one or more stimulation parameters by:
receiving, by way of the one or more interface components, an audio signal included in the one or more audio signals from one of the one or more auxiliary audio input devices; and
transmitting data representative of the received audio signal to the primary sound processor.

9. The system of claim 8, wherein the primary sound processor is configured to generate one or more stimulation parameters associated with the received audio signal in accordance with the transmitted data.

10. The system of claim 8, wherein the one or more auxiliary audio input devices comprise at least one of an FM transmitter, a Bluetooth device, and an audio player.

11. The system of claim 1, wherein the sound processor module further comprises one or more interface components configured to facilitate communication by the secondary sound processor with at least one of a hearing instrument body area network device, an audio gateway device, a remote control device, and another third sound processor.

12. The system of claim 1, wherein:
the sound processor module further comprises one or more interface components configured to facilitate communication by the secondary sound processor with a fitting device; and
the secondary sound processor is further configured to relay fitting data received from the fitting device to the primary sound processor.

13. The system of claim 1, wherein
the sound processor module is communicatively coupled to a loudspeaker; and
the secondary sound processor is further configured to direct the loudspeaker to apply acoustic stimulation representative of at least one of the one or more audio signals to the patient.

## Patentansprüche

1. System mit:
einem Kopfstückmodul, welches ausgebildet ist, um am Kopf eines Patienten befestigt zu werden und einem Primärschallprozessor, welcher darin angeordnet ist und ausgebildet, um Stimulationsparameter zu erzeugen, die verwendet werden, um eine innerhalb des Patienten implantierte Hörprothese dazu zu veranlassen, den Patienten mit elektrischer Stimulation zu beaufschlagen, die repräsentativ für mindestens ein Audiosignal ist, und eine Batterie aufweist, wobei das Kopfstückmodul eine Kommunikationsschaltung aufnimmt, die drahtlos kommunikativ mit einer Kommunikationsschaltung gekoppelt ist, die innerhalb der Hörprothese beinhaltet ist; und
einem Schallprozessormodul, welches von dem Kopfstückmodul getrennt ist und ausgebildet ist, um selektiv und kommunikativ mit dem Kopfstückmodul gekoppelt zu werden und einen Sekundärschallprozessor aufweist, der ausgebildet ist, um
eine kommunikative Kopplung zwischen dem Schallprozessormodul und dem Kopfstückmodul zu erfassen, und
zu der Erzeugung von mindestens einem Stimulationsparameter, der in den Stimulationsparametern beinhaltet ist, beizutragen, während das Schallprozessormodul mit dem Kopfstückmodul kommunikativ gekoppelt ist,
wobei der Primärschallprozessor ausgebildet ist, um
zu erfassen, dass das Schallprozessormodul nicht mit dem Kopfstückmodul kommunikativ gekoppelt ist; und
die Stimulationsparameter unabhängig von dem Sekundärschallprozessor zu erzeugen, während das Schallprozessormodul nicht mit dem Kopfstückmodul kommunikativ gekoppelt ist,
**dadurch gekennzeichnet, dass**
der Primärschallprozessor ausgebildet ist, um den Sekundärschallprozessor mit Betriebsstrom zu versorgen, während das Schallprozessormodul mit dem Kopfstückmodul kommunikativ gekoppelt ist.

2. System gemäß Anspruch 1, wobei der Primärschallprozessor ferner ausgebildet ist, um mindestens einen der Stimulationsparameter unabhängig von dem Sekundärschallprozessor zu erzeugen, während das Schallprozessormodul mit dem Kopfslückmodul kommunikativ gekoppelt ist.

3. System gemäß Anspruch 1, wobei das Schallprozessormodul mindestens einen Nutzereingabemechanismus aufweist, und wobei der Sekundärschallprozessor ausgebildet ist, um zu der Erzeugung des mindestens einen Stimulationsparameter beizutragen, indem
die Interaktion eines Nutzers mit dem mindestens einen Nutzereingabemechanismus erfasst wird; und
der mindestens eine Stimulationsparameter als Antwort auf die Interaktion des Nutzers mit dem mindestens einen Eingabemechanismus eingestellt wird.

4. System gemäß Anspruch 3, wobei der mindestens eine Nutzereingabemechanismus einen Programmauswahlmechanismus, einen Lautstärkesteuerungsmechanismus und/oder einen Empfindlichkeitssteuermechanismus aufweist.

5. System gemäß Anspruch 1, wobei der mindestens eine Stimulationsparameter mit einem in dem mindestens einen Audiosignal eingeschlossenen Audiosignal assoziiert ist, und wobei der Sekundärschallprozessor ausgebildet ist, um zu der Erzeugung des mindestens einen Stimulationsparameters beizutragen, indem:
das Audiosignal erfasst wird;
mindestens eine Vorverarbeitungsfunktion auf das Audiosignal angewandt wird; und
Daten zu dem Primärschallprozessor gesendet werden, die repräsentativ für das vorverarbeitete Audiosignal sind.

6. System gemäß Anspruch 5, wobei das Schallprozessormodul mit mindestens einem Mikrofon kommunikativ gekoppelt ist und wobei der Sekundärschallprozessor ausgebildet ist, um das Audiosignal unter Verwendung des mindestens einen Mikrofons zu erfassen.

7. System gemäß Anspruch 6, wobei die mindestens eine Vorverarbeitungsfunktion eine Beamforming-Funktion aufweist, die mit der Erfassung der Audiosignale unter Verwendung des mindestens einen Mikrofons assoziiert ist.

8. System gemäß Anspruch 1, wobei das Schallprozessormodul ferner mindestens eine Schnittstellenkomponente aufweist, die ausgebildet ist, um die Kommunikation mittels des Sekundärschallprozessors mit mindestens einem Hilfsaudioeingabegerät zu ermöglichen und wobei der Sekundärschallprozessor ausgebildet ist, um zu der Erzeugung des mindestens einen Stimulationsparameters beizutragen, indem:
mittels der mindestens einen Schnittstellenkomponente ein in dem mindestens einen Audiosignal enthaltenes Audiosignal von dem mindestens einen Hilfsaudioeingabegerät empfangen wird; und
Daten an den Primärschallprozessor gesendet werden, die repräsentativ für das empfangene Audiosignal sind.

9. System gemäß Anspruch 8, wobei der Primärschallprozessor ausgebildet ist, um mindestens einen Stimulationsparameter gemäß den gesendeten Daten zu erzeugen, der mit dem empfangenen Audiosignal assoziiert ist.

10. System gemäß Anspruch 8, wobei es sich bei dem mindestens einen Hilfsaudioeingabegerät um einen FM-Sender, ein Bluetooth-Gerät und/oder ein Audioabspielgerät handelt.

11. System gemäß Anspruch 1, wobei das Schallprozessormodul ferner mindestens eine Schnittstellenkomponente aufweist, die ausgebildet ist, um eine Kommunikation des Sekundärschallprozessors mit einem Hörgerät-Körperflächennetzwerk-Gerät, einem Audioverteilergerät, einer Fernbedienung und/oder einem dritten Schallprozessor zu ermöglichen.

12. System gemäß Anspruch 1, wobei:
das Schallprozessormodul ferner mindestens eine Schnittstellenkomponente aufweist, die ausgebildet ist, um eine Kommunikation des Sekundärschallprozessors mit einem Fitting-Gerät zu ermöglichen; und
der Sekundärschallprozessor ferner ausgebildet ist, um von dem Fitting-Gerät empfangene Fitting-Daten an den Primärschallprozessor weiterzuleiten.

13. System gemäß Anspruch 1, wobei:
das Schallprozessormodul kommunikativ mit einem Lautsprecher gekoppelt ist; und
der Sekundärschallprozessor ferner ausgebildet ist, um den Lautsprecher zu veranlassen, den Patienten mit akustischer Stimulation zu beaufschlagen, die repräsentativ für mindestens eines des mindestens einen Audiosignals ist.

## Revendications

1. Système comprenant :
un module de casque configuré pour être fixé à la tête d'un patient et comprenant un processeur de son principal y étant disposé qui est configuré pour générer des paramètres de stimulation utilisés pour conduire une prothèse auditive implantée dans le patient à appliquer une stimulation électrique représentative d'un ou plusieurs signaux audio au patient, et une batterie, le module de casque logeant des circuits de communication qui sont couplés, sans fil, de manière communicative à des circuits de communication contenus à l'intérieur de la prothèse auditive ; et
un module de processeur de son qui est séparé du module de casque et qui est configuré pour être couplé de manière sélective et communicative au module de casque, le module de processeur de son comprenant un processeur de son secondaire configuré pour
détecter un couplage de communication du module de processeur de son avec le module de casque, et
contribuer à la génération d'un ou plusieurs paramètres de stimulation inclus dans les paramètres de stimulation pendant que le module de processeur de son est couplé de manière communicative au module de casque,
où le processeur de son principal est configuré pour détecter que le module de processeur de son n'est pas couplé de manière communicative au module de casque ; et
générer les paramètres de stimulation indépendants du processeur de son secondaire tandis que le module de processeur de son n'est pas couplé de manière communicative au module de casque,
**caractérisé en ce que** le processeur de son principal est configuré pour fournir, pendant que le module de processeur de son est couplé de manière communicative au module de casque, de la puissance de fonctionnement au processeur de son secondaire.

2. Système selon la revendication 1, dans lequel le processeur de son principal est en outre configuré pour générer un ou plusieurs des paramètres de stimulation indépendants du processeur de son secondaire pendant que le module de processeur de son est couplé de manière communicative au module de casque.

3. Système selon la revendication 1, dans lequel le module de processeur de son comprend un ou plusieurs mécanismes d'entrée d'utilisateur, et dans lequel le processeur de son secondaire est configuré pour contribuer à la génération d'un ou plusieurs paramètres de stimulation
en détectant l'interaction d'un utilisateur avec le ou les mécanismes d'entrée d'utilisateur ; et
en ajustant le ou les paramètres de stimulation en réponse à l'interaction de l'utilisateur avec le ou les mécanismes d'entrée d'utilisateur.

4. Système selon la revendication 3, dans lequel le ou les mécanismes d'entrée d'utilisateur comprennent au moins un mécanisme parmi un mécanisme de sélection de programme, un mécanisme de réglage du volume, et un mécanisme de réglage de la sensibilité.

5. Système selon la revendication 1, dans lequel le ou les paramètres de stimulation sont associés à un signal audio inclus dans le ou les signaux audio, et dans lequel le processeur de son secondaire est configuré pour contribuer à la génération du ou des paramètres de stimulation :
en détectant le signal audio ;
en appliquant une ou plusieurs fonctions de prétraitement au signal audio ; et
en transmettant des données représentatives du signal audio prétraité au processeur de son principal.

6. Système selon la revendication 5, dans lequel le module de processeur de son est couplé de manière communicative à un ou plusieurs microphones, et dans lequel le processeur de son secondaire est configuré pour détecter le signal audio au moyen du ou des microphones.

7. Système selon la revendication 6, dans lequel la ou les fonctions de prétraitement comprennent une fonction de formation de faisceau associée à la détection des signaux audio au moyen du ou des microphones.

8. Système selon la revendication 1, dans lequel le module de processeur de son comprend en outre un ou plusieurs composants d'interface configurés pour faciliter la communication par le processeur de son secondaire avec un ou plusieurs dispositifs d'entrée audio auxiliaires, et dans lequel le processeur de son secondaire est configuré pour contribuer à la génération du ou des paramètres de stimulation :
en recevant, par l'intermédiaire du ou des composants d'interface, un signal audio inclus dans le ou les signaux audio à partir du ou des dispositifs d'entrée audio auxiliaires ; et
en transmettant des données représentatives du signal audio reçu au processeur de son principal.

9. Système selon la revendication 8, dans lequel le processeur de son principal est configuré pour générer un ou plusieurs paramètres de stimulation associés au signal audio reçu conformément aux données transmises.

10. Système selon la revendication 8, dans lequel le ou les dispositifs d'entrée audio auxiliaires comprennent au moins un dispositif parmi un émetteur FM, un dispositif Bluetooth et un lecteur audio.

11. Système selon la revendication 1, dans lequel le module de processeur de son comprend en outre un ou plusieurs composants d'interface configurés pour faciliter la communication par le processeur de son secondaire avec au moins un dispositif parmi un dispositif de réseau corporel d'appareil auditif, un dispositif de passerelle audio, une télécommande et un autre troisième processeur de son.

12. Système selon la revendication 1, dans lequel :
le module de processeur de son comprend en outre un ou plusieurs composants d'interface configurés pour faciliter la communication par le processeur de son secondaire avec un dispositif de pose ; et
le processeur de son secondaire est en outre configuré pour relayer les données de pose reçues du dispositif de pose vers le processeur de son principal.

13. Système selon la revendication 1, dans lequel
le module de processeur de son est couplé de manière communicative à un haut-parleur ; et
le processeur de son secondaire est en outre configuré pour conduire le haut-parleur à appliquer une stimulation acoustique représentative d'au moins un du ou des signaux audio au patient.
